# EUROPEAN PATENT APPLICATION

(11) **EP 3 537 351 A1**
(43) Date of publication of application: **11.09.2019**
(21) Application number: 19153606.9
(22) Date of filing: 24.01.2019
(51) Int. Cl.: G06Q 10/06, A61B 5/00, G08B 21/00

(54) **MONITORING DEVICE**

(30) Priority: 09.03.2018 PL 42482318; 14.09.2018 EP 18194669
(71) Applicant: Smart Textiles Sp. z o.o, 00-580 Warszawa (PL)
(72) Inventor: Futera, Konrad, 06-400 Ciechanow (PL); Weinar, Remigiusz, 05-500 Wolomin (PL)
(74) Representative: AOMB Polska Sp. z.o.o.

(57) **Abstract**

A method of monitoring the condition of people, implemented in a system comprising a central server and devices carried by individuals, where devices carried by individuals have power means, logic system, memory, accelerator, preferably multi-axis, and a housing characterized in that measurement of parameters and/or location of the human body on which the device is carried is performed and sent to the central server.

The device is a wearable device to be carried by the user and further comprises an accelerometer, thermometer, magnetometer, blood pressure sensor, microphone, speaker and communicates via wireless using Wifi or Bluettoth or BLE. User's location is determined using a beacon system.

## Description

### Description of the field of the invention

The invention relates to monitoring the condition of people, in particular a remote monitoring of the condition and activity of employees, using a system of distributed location markers and an electronic device carried by an individual.

### Prior Art

The need to monitor different types of people or individuals, in particular to monitor employees in the context of the duties entrusted to them, taking into account their location and involvement in a given period, is the result of observation of employees:
a) Working in groups in a work environment, where it is not possible to directly evaluate the tasks entrusted to a person or it requires time and using additional resources, including people - e.g. a construction team, a gas station or supermarket or restaurant team, factory crew
b) Working individually in a work environment, where it is not possible to directly evaluate the tasks entrusted to a person or it requires time and using additional resources, including people - for example - cleaning company employee at the hotel, security guard at the construction site.

Often employees of construction, production or service companies perform their tasks in places where it is not possible of observe them, in a continuous manner, often in groups responsible for the joint performance of the task. In such cases determining if a given employee is really performing its duties and if and how long he stays at the place where he should perform his duties, is valuable information for the person assessing the quality of work of a given employee.

The issues raised concern, therefore, all those employees who perform physical work or such that involves a change of location as part of their duties. The need for analysis and optimization of processes affecting the cost of labour will grow due to several factors:
a. Rising wage costs - increasing employee expectations;
b. Increasing costs related to wages, directly proportionally increasing along with an increase in wages and salaries;
c. Increasing alternative costs resulting from rising labour costs;
d. In the case of non-compliance with health and safety - costs of removing the effects of undesired or unforeseen events and costs of random events.

A case of a Polish discounter is known, which ordered employees to wear devices recording the work of the heart using the Holter method. The data thus obtained could then be used to draw conclusions about the activity of employees, in particular their physical activity during the day, which in turn could reflect the performance of duties by employees, the degree of involvement in work, etc. The example cited, however raised controversy regarding the potential threatening of employees' personal rights in connection with which the project was interrupted - clearly shows interest in the subject and an appropriate business need on the part of employers, not only in the commercial chain industry, but in many different industries, including security, construction, transport, medical care, elderly or children agencies, hotels, etc.

Various types of devices and systems for monitoring the location and the physical condition of the persons wearing electronic devices are known in the prior art . For example, a system known as Endomondo is commonly known, which uses a smartphone carried by a person during physical activity. Thanks to the sensors in the phone, such as GPS and accelerometer, it is possible, e.g. to record the runner's route, speed of the run along the entire route as well as to detect temporary maximum/minimum speed, as well as to count steps. Based on this data, a computer program is able to calculate, among others, the predicted amount of calories burned, which is particularly useful for people on a diet. Unfortunately, the use of the phone may not be suitable for industrial applications, to monitor the activity of employees, as such applications usually require a good cellular range, and the phone consumes more energy during this mode of operation than in a standby mode, so there is a risk that the use of Endomondo and smartphone applications to monitor the status of employees would be significantly limited in time.

There are also smartwatch devices that, due to the forearm mounting, can measure the user's pulse or even blood pressure, which, e.g. transmitted to a smartphone on a regular basis, enable a more comprehensive collection of information about the health status of the user and the nature of physical activity. Such devices are, however, complicated and therefore expensive and usually have a touch screen, etc. - such devices are not suitable for use on an industrial scale and for a large number of employees, due to their uneconomicality and insufficient durability.

Similarly to smartwatches, the prior art , e.g. the application US20180025605A1, discloses a bracelet that has a built-in GPS module and is primarily used to quickly find lost people, especially children. However, this type of solution will not help in collecting information about people who are beyond the reach of the cellular network or GPS signal, making the device inappropriate for use, e.g. in factories.

On the other hand, the application CA2717866A1 discloses a system for monitoring individuals in which arranged beacons are used (small devices using, e.g. Bluetooth protocol to send their ID number in the immediate vicinity), while a monitored individual wears a device that records the presence of a given beacon, and then it is able to wirelessly send information about the location of the individual to the central office (i.e. the collected beacons' ID along with the time mark, indicating that it has found a given beacon in the given time). This solution, however, does not provide for registration of the health condition or form of physical activity of a person wearing a device cooperating with beacons.

### Technical problem and object of the invention

The existing employee monitoring systems boil down to video monitoring only, which means that in order to get any data about work performed by a human being, one should always look at his work. This matter is becoming significantly complicated if we have more than one person to watch. It is not difficult to imagine a group of five construction workers performing a task related to the construction of a house, where one of the workers has a tendency to abuse alcohol. It can be assumed that if one worker abuses alcohol, then:
- will be less productive than others (and therefore will receive the same pay despite the lack of work, will worsen the work of the whole team, who will see that this behaviour takes place)
- can create a threat with his behaviour or some omission (it is possible that this worker being under the influence of alcohol will not turn off the device when leaving the workplace, which may in extreme cases result in overheating the device and causing a fire).

In view of the above, there is a need for systems and methods that would enable the use of low-cost and simple devices to monitor the status of employees, both in large industrial plants and in less extensive workplaces, in order to respond quickly to health and life hazards, but also to evaluate the behaviour of employees during the working day.

The described need can be effectively satisfied by using the present invention.

The invention relates to a method of monitoring the condition of people, implemented in a system comprising a central server and devices carried by individuals, where devices carried by individuals have power means, logic system, memory, accelerator, preferably multi-axis, and a housing characterized in that they include the following steps:
a. measurement of the acceleration of the part of the human body on which the device is carried by an individual by means of an accelerometer;
b. recording the results of this measurement by means of the logic system in the memory of the device carried by man;
c. sending data stored in the device's memory from the device carried by a man to the central server;
d. analysing the data referred to in step c, especially performed by the central server, in terms of detecting occurring anomalies, in particular periods with accelerations lower or higher than expected or without any registered accelerations, preferably by comparing them with reference data.

Preferably, the system further comprises one or more beacons dividing the physical space into zones, wherein the device carried by an individual has means to communicate with the beacons and to record the time and identifier of said beacons in the range of which there is the device carried by an individual, wherein
the memory of the device carried by an individual, records the time and the identifier of the beacon, with which the device carried by an individual communicated;
data related to the time and identifier of the beacon with which the device carried by an individual communicated is sent to the central server,
data related to the time and identifiers of the beacons with which the device carried by an individual communicated is analysed, to detect anomalies, in particular longer and shorter than expected periods of presence in a particular zone, as well as presence of an individual in a zone in which they should not be present.

Preferably, the system further comprises means for wireless communication, preferably of WiFi, Bluetooth or BLE type, in particular routers or signal amplifiers connected to the central server, wherein the device carried by an individual has means for wireless communication with means for wireless communication, preferably of WiFi, Bluetooth or BLE type, wherein
the device carried by an individual sends wireless data to the central server;
the data sent from the device carried by an individual is analysed to detect anomalies.

Preferably, the device carried by an individual is used having an additional magnetometer, for detecting the setting of the device carried by an individual relative to the earth's magnetic field, wherein
the data concerning the position of an individual relative to the earth's magnetic field measured with said magnetometer is recorded in the memory of the device carried by the user,
these data is analysed for the anomaly of the individual position.

Preferably, the logic is used included in a device carried by an individual and/or a central server containing a set of predefined ranges of value of qualities measured by a device carried by an individual, in particular responding to threat situations, especially accelerations, upon detection of which the central server generates alarm information.

Preferably, the device carried by an individual is used having an additional thermometer, wherein
the temperature of body or human environment measured with said thermometer is recorded,
said data is analysed for anomalies regarding measured body or human environment temperatures.

Preferably, the device carried by an individual is used having an additional pressure sensor, wherein
the ambient pressure, blood pressure and/or human pulse measured with said sensor are recorded in the device's memory,
said data is analysed for anomalies regarding measured ambient pressure, blood pressure and/or human pulse.

Preferably, the device carried by an individual is used having an additional microphone and loudspeaker and a user interface, preferably in the form of a button, where the logic system of the device is capable of processing voice conversations by means of the wireless communication elements present in the device, where the user interface can, preferably, be initiated or ended by the voice call, wherein
if an anomaly is detected that could indicate a hazardous situation as a result of data analysis in any of the measurements sent to the central server by the device carried by an individual, a voice call is initiated with a person in whom the possible threat has been detected .

Preferably, the condition of people being employees is monitored, in particular store employees, warehouse employees, security guards, rehabilitators, elderly guardians, policemen, factory workers, especially with belt production lines.

The invention also relates to a system for monitoring the condition of people for implementing a method for monitoring the condition of people according to any one of the preceding claims, characterized in that
a. at least one device carried by an individual, comprising power means, logic system, memory, accelerator, preferably multi-axis, and a housing
b. a central server, preferably a computer, having means to retrieve data from a device carried by an individual,
wherein the device carried by an individual is capable of record the data by the sensors that are part of the device carried by an individual, in particular the accelerometer, and
the central server is capable of analysing data retrieved from the device carried by an individual, in particular by comparing it with reference data to detect anomalies and further disclose information about the occurrence and nature of the anomaly detected.

Preferably, the system further comprises one or more beacons dividing the physical space into zones, wherein the device carried by an individual has means to communicate with the beacons and to record the time and identifier of said beacons in the range of which there is device carried by an individual.

Preferably, the system further comprises means for wireless communication, preferably of WiFi, Bluetooth or BLE type, in particular routers or signal amplifiers connected to the central server, wherein the device carried by an individual has means for wireless communication with means for wireless communication, preferably of WiFi, Bluetooth or BLE type.

Preferably, the device carried by an individual additionally has one or more of the following devices: a magnetometer for detecting the setting of the device carried by an individual relative to the earth's magnetic field; a thermometer for measuring of temperature of body or human environment; pressure sensor for measuring the ambient pressure, human pulse and/or blood pressure; microphone and loudspeaker and a user interface, preferably in the form of a button, where the logic system of the device is capable of processing voice conversations using the wireless communication elements present in the device, where the user interface can preferably be initiated or ended by the voice call.

Preferably, the logic system included in a device carried by an individual and/or a central server contain a set of predefined ranges of value of qualities measured by a device carried by an individual, in particular responding to threat situations, especially accelerations, upon detection of which the central server may generate alarm information.

Preferably, the device carried by an individual has a substantially cuboidal housing and has seven buttons, of which:
- six buttons (5, 25) are placed in two groups of three placed in one line of the buttons (5, 25), near and along the two shorter opposite edges of the front surface of the housing (1, 21) of the device, at regular intervals, wherein the middle buttons in each group of buttons (5, 25) being at the same distance from the longer opposite edges of the front surface of the housing (1, 21) of the device
- the control button (6, 26) is placed on the front surface of the housing (1, 21) at the symmetry centre of the rectangle defined by the shorter side wall of the housing (1, 21) and the adjacent halves of the longer side walls of the housing (1, 21),
wherein the buttons (5, 25) resist pressing against an equivalent mass force of 150 g to 500 g, preferably 300 g,
the signalling diode (3, 23) is placed in the symmetry centre of the rectangle defined by the left longer side wall of the housing (1, 21) and the adjacent halves of the shorter side walls of the housing (1, 21).

### Description of drawings

The invention will now be described in greater detail in preferred embodiments, with references to the attached drawings, in which:
Fig. 1 shows an embodiment of the device with a button arrangement at the front of the device housing,
Fig. 2 shows another embodiment of the device with a button arrangement on the perimeter of the housing, and
Fig. 3 shows an example diagram of the system for monitoring the condition of people.

### Preferred embodiments

The embodiment of the invention described below relates to a method and system for wireless monitoring of employees, where the main aspects which the employer would like to pay attention to are:
1. Monitoring "the involvement" of the employees in performed duties and their suitability to perform a said range of activities based on "physical activity" of employees in the performance of a given type of tasks - by comparing the reference case (e.g. optimal) with the measured one.
2. Monitoring of the exemplary zones in which individual employees are present at a given time in a given type of work:
   a. The restaurant (consumption room, hall, reception, kitchen, warehouse)
   b. Shop (sales hall, facilities, supply zone, social zone)
   c. Gas station (cash register, commercial and catering part, distributors, deliveries)
   d. Office building (floor X, surface Y)
   e. Hotel (X room, Y floor, lobby, kitchen, warehouse and social part, corridor Z)
   f. Section of the construction of a linear structure being carried out, e.g. a highway (kilometre XYZ)
   g. Hazardous waste treatment plant (ABC zone, safe, danger zone)

In order to ensure the monitoring of employees in the manner described above (or analogous, for workplaces not mentioned above), it is necessary to prepare technical means and combine them into a system that will be able to implement the method according to the invention.

First, it is necessary to provide a device that would be carried by an individual, i.e. a person being monitored, and which device would act as a sensor. Such a device may preferably have:
- an accelerator, preferably multi-axis one,
- magnetometer,
- elements for wireless communication, preferably WiFi and according to various types of Bluetooth protocols,
- elements for communication with beacons,
- solid housing,
- thermometer,
- pressure sensor,
- loudspeaker and microphone,
- one or a few buttons,
- location module, e.g. GPS or using the GSM network,
- internal power supply and possibly charging module,
- logic system controlling the components of the device.

In addition to the device carried by an individual, it is necessary to provide auxiliary devices, which can include:
- beacons,
- routers and other devices for communication/intermediation in wireless communication, at least one of which is connected to the Internet or local area network,
- central server, e.g. a computer with access to the Internet or local area network,
- cameras,
- loudspeakers and microphones,
- drones.

The role of beacons, or relatively simple devices, emitting a wireless signal containing the identifier (ID) of a given beacon in their immediate vicinity, is the marking of zones in space, e.g. work zones. A person with a device capable of reading the beacon ID and sending this information, e.g. via a WiFi network to the central server, is registered as one that appeared in the given environment. For example, a device for a person cleaning the hotel entering the floor Y - records the beacon ID placed at the entrance to the floor Y, and then - when that person enters the room X - records the beacon ID placed at the entrance to the room X, then registers the beacon ID by room X again when the person finished cleaning room X and comes out of it. Another example is a set of beacons placed in an industrial plant, with numerous machines (e.g. lathes) and a canteen. If an employee approaches a lathe at which he should perform the ordered work, the device carried by an individual registers the beacon ID placed near the lathe. When the employee then goes on a break, the beacon ID will be registered at the entrance to the canteen. When the employee returns to the workplace, the beacon ID placed at the entrance to the canteen and at the work station, e.g. at the lathe, should be recorded in sequence.

There are many possibilities to deploy beacons in the area where the work is done, and the simplicity of beacons is to be able to secure such beacons in a lot of places at a low cost, thus obtaining comprehensive knowledge about the time employees stay in the zones. Moreover, minimizing the beacon function can also be used to mount them in places where it would be difficult or inadvisable to communicate with the central server via wired or wireless connections. Employees, in turn, move around the workplace, so ID of beacons secured in places with limited communication with the rest of the system can be recorded in a device carried by an individual and sent to a central server, e.g. at the first opportunity, when an employee enters the range of the company's WiFi network.

Another preferred effect of using beacons is the ability to collect data on, e.g. the speed and direction in which the employee moves. For example, if two beacons are mounted on both sides of the door, the device carried by an individual will first register the ID of one of these beacons, and then the second one - one can find out in which direction the employee is going through such a door. When mounting several beacons along the corridor in the factory hall, based on the time of recording the ID of subsequent beacons, it is possible to calculate the speed of the individual movement and, e.g., verify whether the employee goes at the normal pace or runs despite a clear ban on running in the factory hall.

Another information that can be obtained with the help of beacons is the time spent in a given zone. For example, if an employee operates a lathe and according to the work schedule and health and safety regulations should go on a 10-minute break every 60 minutes, the central server should record the beacons ID at the lathe and, e.g. the canteen at more or less such intervals. If, however, the ID of the beacon is first recorded by the lathe, after the hour of the beacon by the canteen and after two hours the beacon's ID is registered again by the lathe, this may indicate a much longer break at work - which may be caused by laziness or worse health condition - in each case, the supervisor would want to find out and talk with the employee to explain his behaviour. On the other hand, when after starting work by the example employee discussed above and recording the beacon ID by the lathe at the beginning of the day - there is no registration of the beacon ID by the canteen for 8 hours - this may indicate that a given person has too much work, which in turn could also require intervention of the supervisor, so that the overworked employee's health did not get worse and as a result of fatigue did not cause an accident or had an accident.

On the other hand, routers and other devices for wireless communication or ordinary signal transmission are used to allow, when placed in the work area, to transfer the data from devices carried by employees to the central server. There are many different configurations possible, i.e. exemplary routers can cover the entire work area (performing continuous monitoring, e.g. in workplaces particularly exposed to danger) or can be placed only at the entrances to the work area (to collect information from the whole day, e.g. in places with a lower risk of accident or in places where the presence of a wireless network is undesirable due to possible interference of operating devices, etc.).

The central server is preferably a computer with access to the Internet or a local network that collects information sent by devices carried by employees and has software for analysing this information - e.g. comparing reference results and results recorded by devices carried by employees. Such software on the central server can then generate messages for people supervising the work of employees, whether the work was according to the expected scheme or maybe there were anomalies that may require explanation.

In turn, cameras, loudspeakers and microphones can be used as additional tools for monitoring employees, where their use (e.g. directing the camera in a given direction, calling a specific person through a loudspeaker to transmit information via a microphone, etc.) can be initiated from the central server after detecting anomalies in human behaviour, based on information sent by the device carried by an individual. Drones (flying, riding or even flying under or on the surface of the water) can be used for a similar purpose, in particularly large areas of work, e.g. on construction sites, bridges, etc., where installation of cameras, loudspeakers may be limited and it may be necessary to quickly find a given person.

The device carried by an individual may have a form suitable for a given profession, e.g. if it is an employee who has a pocket in his outfit - it may be a device that fits in such a pocket. If the employee does not have the pockets or it is inadvisable to have such a device in the pockets - it may have own elements (housing elements) or additional (e.g. pouch, detachable straps, velcro) that will allow the device to be attached to a man's clothing or belt. In yet another configuration (e.g., when the device would collect information about the human pulse) it would be beneficial if the device was attached to the human body, e.g. to the hand or leg (i.e., the device would have a form similar to a wristband or watch). Moreover, it is preferred that the device may remain on the premises of the workplace (e.g. to prevent employees from interfering with the internal components of the device), i.e. any possible means of securing the device to the clothes or human body enable putting down the device after the work has been completed.

An important element of the device carried by an individual is the accelerometer, preferably multi-axis one, which will enable continuous measurement of the acceleration of the part of the human body by which the device carried by an individual will be. Acceleration measurements, in particular in many axes, can provide a lot of information about the human condition - e.g. a complete lack of detected accelerations can indicate that an employee lost consciousness or fell asleep; regular and moderate (i.e. in the expected range) acceleration may be indicative of a regular work of a given person (e.g. a bricklayer or a person operating a lathe); sudden accelerations above the expected level may indicate a rush, quarrels, a fight, an epilepsy attack, and when they occur one at a time - about collision, fall from height or other sudden event. Using an accelerometer, as required, the device can be programmed to collect information continuously (thus creating a database of accelerations from the entire working day) or only when the accelerometer's readings go beyond previously assumed values (registering only anomalies in human behaviour).

The next preferred element of the device carried by an individual is the magnetometer, which in particular in the case of people working in places where the Earth's magnetic field is detectable, can give information about which direction the employee is turned at the moment. In addition to the Earth's magnetic field, it is also possible, in addition to the magnetic field in a device carried by an individual, to install magnetic markers at the workplace, which would be detected by the magnetometer, also serving the purpose of determining the direction of human turning or movement. This can be particularly preferred in cases where the workplace has access only from one side (e.g. a drafting table, desk placed next to the wall, cash register in the store, etc.) and the employee often turns around for a long time (i.e. stops working). While obtaining information about the direction of turning of a person at a given moment could be difficult using only accelerometers and beacons, the magnetometer is suitable for this purpose. Moreover, if a person moves, e.g. around the construction site, but goes backwards - can be particularly exposed to danger (protruding parts, passing vehicles, etc.) - the magnetometer readings for the turn can be helpful to detect people who have bad habits and instruct them about the need for more attention.

Another, almost essential element of the device carried by man, are means for wireless communication, e.g. via WiFi or various types of Bluetooth protocols. Thanks to such measures, a device carried by a human can send information to the central server during the working day. It is possible to use a device that does not have means for wireless communication and information from the work day would be transmitted, e.g. by connecting the device to a central server via a cable - such devices to be carried by employees can be particularly preferred in workplaces where wireless networks are not recommended. However, many establishments and workplaces do not have such limitations, which in the vast majority of cases makes it beneficial for a human-carried device to have the means to communicate in such a way as to be able to send information to the central server on a daily basis and enable quick detection of anomalies in the workplace. employees' behaviour and quick reaction of supervisors.

Another important element of the device carried by man is the means of communication with the beacons, i.e. enabling the reading and registration of the beacons' ID, near which the employee will be found. Depending on the type of beacons used, the aforementioned means of wireless communication (e.g. WiFi or Bluetooth) may serve this purpose, but they may also be other communication modules, adapted, e.g., only for communication with beacons.

The device to be carried by an individual, having at least the elements discussed above, needs a logic system which, according to pre-defined procedural protocols in its memory, would coordinate the operation of device components and communication with external elements, such as beacons or a wireless network present in the workplace. Extensive memory recording measurements from the device's sensors may turn out to an important element of the logic system, in particular if the device would not send or delete the measurement results on a regular basis, but collect data throughout the day and transfer them to the central server only after completion of work. Depending on the amount of all elements of the device carried by an individual and the desired possible interaction with the device (i.e. whether the device only collects data and has no user interface in any form or has buttons, microphone and speaker and can be used as a device for voice communication) the logic system can be simple or more complicated and with more possibilities.

Another essential element of the device carried by an individual is the internal supply module of the device, preferably in the form of an accumulator. Such a supply module should enable the device to operate for at least the scheduled duration of work in one day, e.g. for eight hours or more - to reduce the number of device charging cycles, which cycles can shorten the lifetime and performance of e.g. the accumulator. In order to charge the device, many solutions are possible - the power module can be removable, it can be charged by connecting the power supply to the device or it can be charged wirelessly. Individual places of use of the device carried by an individual may enforce specific constructions, e.g. a worker exposed to frequent contact with water or working in an environment where there are fumes or fine dust should use a device protected against such factors and may preferably have a wireless charging module (with tight housing).

The housing of the device carried by an individual is preferably solid and made so as to effectively protect the elements inside the device. It can be made of plastic, metal, rubber, composites, natural leather, etc. or a combination of such materials. Taking into account the workplace, other requirements may be set for the housing, i.e. for people working outdoors during the day, exposed to long-lasting sunlight, a metal housing may not be suitable - it may overheat and cause burns (e.g. devices carried on the hand). On the other hand, for people working in an environment where there is a lot of moisture, housings made of natural leather (which could eventually be soaked) may be unsuitable. A person skilled in the art would be able to choose the right housing for use in a given work environment, wherein plastic housings appear to be most preferred, e.g. locally covered with a rubber layer. Another aspect of the housing is the way it is closed/opened - the housing of devices that have replaceable batteries should have opening/removable elements (enabling access to the battery), and the housing of devices such as those charged wirelessly for people working in an environment exposed to water should be sealed, closed, e.g. with snaps or screwed.

Due to the possibility of using the invention in many branches of industry, it may be preferred to collect other human data, which data may sometimes be considered too personal, i.e. regarding the current health condition - e.g. body temperature, heart rate or blood pressure. However, for professional groups in which there is a relatively high risk of dangerous situations (e.g. miners, security guards) or for people working outside in winter (who may suffer from frostbite), collecting such data may prove beneficial (through faster detection of e.g. frostbite, anomalies in the heart rhythm, etc.) to be able to react quickly. Therefore, the used device carried by an individual, if it had e.g. a form of the band carried on the forearm in direct contact with the skin, may also have, e.g. a thermometer or pressure sensor measuring these parameters of the human body. In another possible configuration, a pressure sensor or thermometer can be used to detect other environmental anomalies, e.g. when measuring ambient pressure, violent pressure spikes or temperatures indicative of an explosion, fire, etc. can be detected.

In particularly sophisticated configurations of the device carried by an individual, it is also possible to put a loudspeaker and a microphone in the device that would allow the supervisor's voice to interact with the employee.

Most elements of the device carried by an individual work in a predefined and preprogrammed way (e.g. whether measurements are registered continuously, or only anomalies are recorded) and do not have to require interference of the person - as a result, the device carried by an individual may not have a user interface (e.g. in the form of buttons) and have a relatively compact housing. However, if a microphone and a loudspeaker is used, it may be preferred to use, e.g. a button initiating/ending the call. Also in the case of devices for security guards patrolling large objects, it may be preferred to use the interface e.g. in the form of a buzzer, light and/or a button that would call the security guard to contact the control panel (e.g. to verify the reason for the security guard's longer immobility). The user interface can therefore be a part of the device carried by an individual (e.g. in the form of a button, buzzer, LED diode, etc.), but in other cases such an interface may be unnecessary and therefore not present (the device may be a specific "black box").

In the case of applying the invention to people working in a large space, in which it would be difficult to use beacons to mark the places (e.g. a person patrolling a large water body on a boat), it may be preferred if the device carried by an individual had a location module, e.g. a form of a GPS module or a module using the GSM network. In the case of a device connecting to the GSM network it would also be possible to communicate with the server using data transmission via the GSM network. In turn, for devices dedicated for employees who work in industrial halls where beacons will be installed - such GPS or GSM modules can be completely unnecessary.

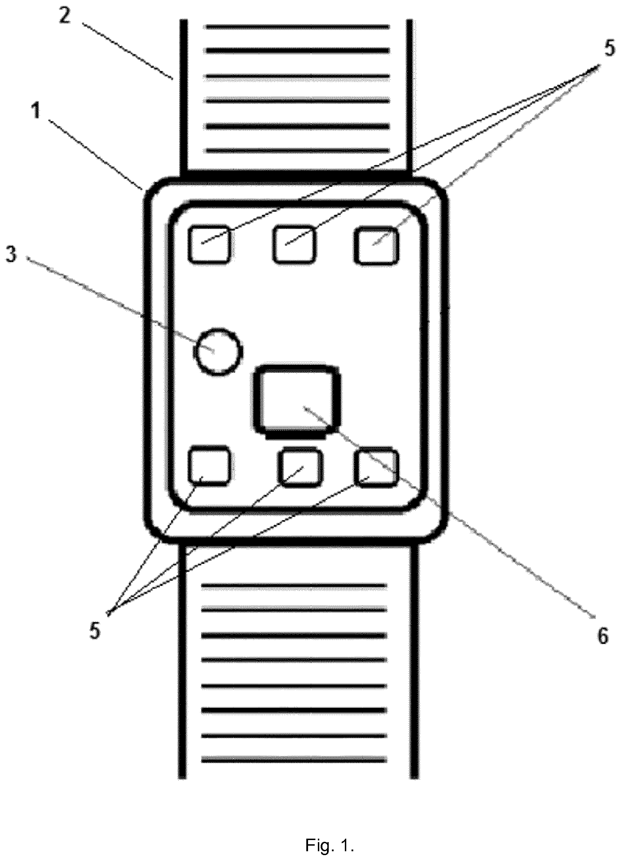

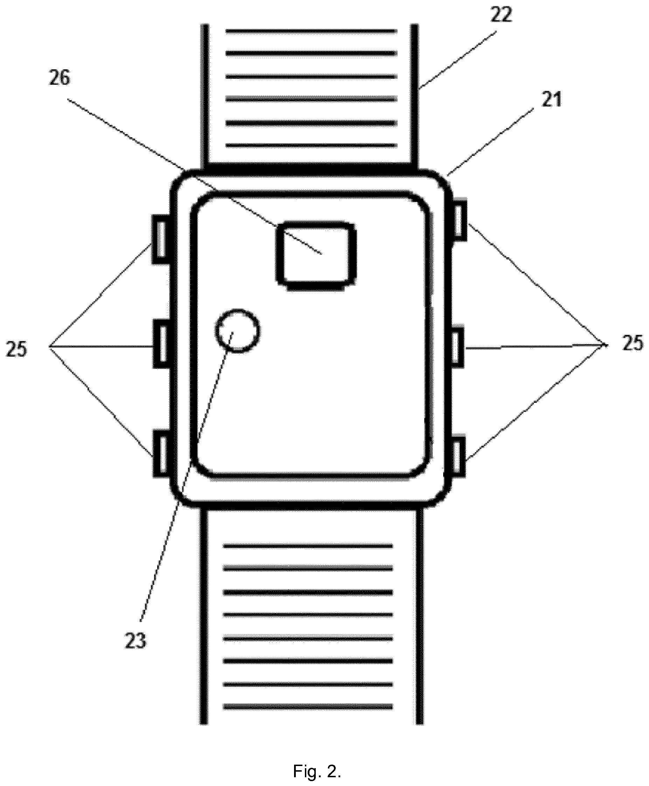

It may also be preferred for the device carried by an individual to have a user interface with more than one button. According to the Polish patent application P-424823 of March 3, 2018 whose priority the current application uses, the structure of the housing being part of the band has proven particularly preferred. In such a preferred embodiment, shown in Fig. 1 and fig. 2 the device carried by an individual is in the form of a substantially cuboidal housing with a strap, e.g. for a forearm attachment, where there are seven buttons on the case, of which:
- six buttons 5, 25 are placed in two groups of three placed in one line of the buttons 5, 25, near and along the two shorter opposite edges of the front surface of the housing 1, 21 of the device, at regular intervals, wherein the middle buttons in each group of buttons 5, 25 being at the same distance from the longer opposite edges of the front surface of the housing 1, 21 of the device
- the control button 6, 26 is placed on the front surface of the housing 1, 21 at the symmetry centre of the rectangle defined by the shorter side wall of the housing 1, 21 and the adjacent halves of the longer side walls of the housing 1, 21, wherein the buttons 5, 25 resist pressing against an equivalent mass force of 150 g to 500 g, preferably 300 g,
- the signalling diode 3, 23 is placed in the symmetry centre of the rectangle defined by the left longer side wall of the housing 1, 21 and the adjacent halves of the shorter side walls of the housing 1, 21.

In a preferred embodiment, the buttons 5, 25 protrude above the surface of the housing 1, 21 less than 1 mm, preferably less than 0.2 mm, most preferably do not protrude above the surface of the housing 1, 21, and the control button 6, 26 protrudes above the surface housing 1 to 3 mm, preferably 2 mm.

With reference to the above description, it is clear that the invention uses some kind of devices (beacons, means of wireless communication, server and devices carried by employees) and is not limited to only one configuration. Moreover, the invention relates to a method of monitoring employees during work, where the working environment is suitably adapted (i.e. a server and devices to be carried by employees is provided and configured accordingly, since additional wireless means and beacons are preferred, but are not required for all possible fields of application) and employees carry devices that collect information on the behaviour of employees during the day (in which zone they are, how intensively they work, whether they move calmly or run, how much time they spend in a given zone), from which during the day or later (e.g. at the end of the day, after handing over the device carried by the supervisor) the data is sent to the central server, which analyses the collected data (e.g. detecting anomalies and comparing these anomalies with probable causes/events/behaviours).

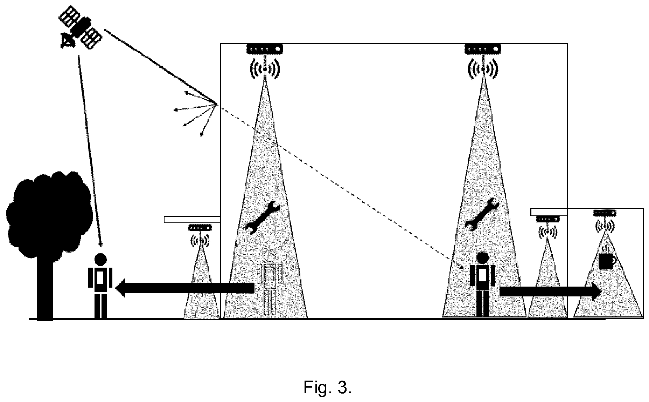

Fig. 3 schematically shows the workplace and key elements of the system for monitoring the condition of people. Symbolic designations of the beacons, that send a signal in a narrow area below each other are visible, although this is not the only possible realization and you can place beacons around the floor (to send a signal in the upward direction, e.g.) or e.g. in the walls (to send signal from the side). Beacons are located in several places - at the exit/entrance to the workplace, at workplaces (marked with keys), at the exit to the factory canteen and at the factory canteen. This arrangement of beacons provides the possibility of precisely defining the area in which the employee is present (by registering by the employee the ID of the particular beacon being carried by the employee at a given moment). Moreover, information about the employee's location, when outside of the workplace, can be collected using the satellites and the GPS module of the device carried by the employee. Fig. 3 shows this through a continuous line connecting the satellite with the employee (meaning effective communication) and a continuous line that passes into a dashed line from the satellite to the employee (meaning the possible direction of connection, but blocked by the walls of the building).

On the other hand, thick arrows show two examples of the employee's mobility. In the first case, the employee leaves the work station (on the left side of the figure) and leaves the building. The device that he carries then first records the beacon ID located above the work station, then the beacon ID located at the entrance to the building, finally outside the building (in this example configuration of the system) there are no more beacons, but the device carried by the employee connects through the GPS module and records the current location of the employee. In the subsequent analysis of the readings of the device carried by the employee, it is possible to detect what time the employee left the building and how long he stayed there. In turn, if the employee leaves on an official bases away from the building of the workplace (e.g. a ranger going to the national park observation), the GPS module of the device carried by the employee records the route and places he visited. In the second possibility of the employee movement (on the right side of the figure), the employee leaves the workplace and goes to the factory canteen (e.g. to eat a meal and drink coffee). The device that he carries will first record the ID of the beacon above the work station, followed by the beacon ID before entering the canteen and finally the beacon in the canteen (and all those mentioned, only in the reverse order when the employee returns to the workplace). During later analysis of the data it will be clear how much time the employee spent in the canteen (based on time marks of recorded beacon IDs), and it will even be possible to assess the rate at which he moved to and from the canteen (distinguishing between a normal situation and the situation in which the employee is in a hurry and runs , against workplace regulations).

Although Fig. 3 shows one configuration of the system for monitoring the condition of people, it is obvious that depending on the type of workplace (both in terms of building features and work performed) there are many different configurations that originate from the same idea, i.e. monitoring the condition of people by measuring body kinematics and collecting data about the place and duration of presence in zones separated by beacons.

The use of the invention can be particularly important in terms of:
a) Occupational health and safety - can increase the safety of employees by obtaining detailed data on the behaviour of employees in the performance of various commissioned works (the ability to analyse data and exclude dangerous behaviours, risky for employees and other people or property);
b) Occupational health and safety - in particular in the context of dangerous professions, where the employee is for some reason staying too long in the area adversely affecting his health e.g. due to temperature, noise, vibrations, radiation or other factors (to detect such situations and early enough to respond);
c) Possibilities of obtaining relevant data and information regarding the determination of possible liability and/or/and man/employer/ principal/third parties/other circumstances in the case of events causing recourse to the employer or man, i.e. events related to property or criminal liability (accidents at work, thefts, insurance damages);
d) The possibility of obtaining data and information allowing confirmation of a specific order by a subcontractor, e.g. a cleaning company in an office or hotel in a specific area and time.

The described configurations of the device carried by an individual can be suitably adapted to specific work environments in which the method of monitoring the employees according to the invention would be used.

For example, one can monitor employees of a rehabilitation centre. Due to the arrangement in the corridors and at the entrances to the rooms (both rooms and exercise rooms, canteen, bathrooms, etc.), it is possible to verify in which areas the employees are staying. In turn, due to motor measurements, it is possible to analyse the involvement of employees during rehabilitation activities. Specifically, if a person's duty is to help in performing the exercises, the device carried by this employee will register a given histogram of physical activity (whether it is the hand on which the device carried by the employee would be placed or the whole body if the device carried by the employee was located in a trouser pocket, e.g.). After analysing such a histogram of activity, it becomes clear whether the person was really attending to his duties and helping rehabilitated people, or, in an extreme case, only led the mentee to the exercise room and sat in a chair without actively helping a rehabilitated person. Also, by analysing the time of presence in the given zones, it will be possible to verify with whom the carer spends what amount of time and in what form - e.g. this results in progress in rehabilitation and less time spent in the exercise room and more time spent in the garden walking.

Another professional group such as police officers can also use such devices. On the basis of GPS position measurements and body movement measurements, the course of their service can be recreated. E.g. when monitoring mass events, it will allow easy identification of the behaviour of police officers - all sudden accelerations will be detected, which will allow to recreate the course of intervention or assault on a given policeman. Also, in the case of daily patrols, it is easy to detect the policeman's activity - whether he walked, whether he rode or chased someone. This allows to verify reports as well as current monitoring of the policeman's health, for example, if his activity without any external cause began to decrease rapidly overnight.

In yet another configuration of the invention, the method of monitoring employees could be adapted to the factory with belt production. The device carried by the employee could be carried both on the body, e.g. in the pocket and on the hand. In this configuration, both the time spent in a given zone in the workplace is measured, to detect anomalies, e.g. excessive stay in the canteen, and (especially if the device is carried on the hand/arm) receive data about the work rate of the employee. Motor data collected in this way is particularly important when it comes to employees who assess the quality of products - when an exceptionally slow, even lazy nature of work is detected, there is a suspicion that the employee did not perform his duties with due diligence and it is good to check specific lots of products once again to avoid the release of defective products from the factory that could endanger the life or health of end consumers.

The invention is equally applicable to monitoring other groups of people - for example hotel guests.

## Claims

1. A method of monitoring the condition of people, implemented in a system comprising a central server and devices carried by individuals, where devices carried by individuals have power means, logic system, memory, accelerator, preferably multi-axis, and a housing **characterized in that** they include the following steps:
a. measurement of the acceleration of the part of the human body on which the device is carried by an individual by means of accelerometer;
b. recording the results of this measurement by means of the logical system in the memory of the device carried by man;
c. sending data stored in the device's memory from the device carried by a man to the central server;
d. analysing the data referred to in step c, especially performed by the central server, for detecting occurring anomalies, in particular periods with accelerations lower or higher than expected or without any registered accelerations, preferably by comparing them with reference data.

2. The method according to claim 1 **characterised in that**
the system further comprises one or more beacons dividing the physical space into zones, wherein the device carried by an individual has means to communicate with the beacons and to record the time and identifier of said beacons in the range of which there is the device carried by an individual, wherein
the memory of the device carried by an individual, records the time and the identifier of the beacon, with which the device carried by an individual communicated;
data related to the time and identifier of the beacon with which the device carried by an individual communicated is sent to the central server,
data related to the time and identifiers of the beacons with which the device carried by an individual communicated is analysed, to detect anomalies, in particular longer and shorter than expected periods of presence in a particular zone, as well as presence of an individual in a zone in which they should not be present.

3. The method according to claim 1 or 2, **characterized in that**
the system further comprises means for wireless communication, preferably of WiFi, Bluetooth or BLE type, in particular routers or signal amplifiers connected to the central server, wherein the device carried by an individual has means for wireless communication with means for wireless communication, preferably of WiFi, Bluetooth or BLE type, wherein the device carried by an individual sends wireless data to the central server;
the data sent from the device carried by an individual is analysed to detect anomalies.

4. The method according to claim 1, 2 or 3, **characterized in that**
the device carried by an individual is used having an additional magnetometer, for detecting the setting of the device carried by an individual relative to the earth's magnetic field, wherein
the data concerning the position of an individual relative to the earth's magnetic field measured with said magnetometer is recorded in the memory of the device carried by the user,
these data is analysed for the anomaly of the individual position.

5. The method according to claim 3 or 4, **characterized in that**
a logical system is used included in a device carried by an individual and/or a central server containing a set of predefined ranges of value of qualities measured by a device carried by an individual, in particular responding to threat situations, especially accelerations, upon detection of which the central server generates alarm information.

6. A method according to any one of claims 1 to 5, **characterized in that** the device carried by an individual is used having an additional thermometer, wherein
the temperature of body or human environment measured with said thermometer is recorded,
said data is analysed for anomalies regarding measured temperatures of body or human environment.

7. A method according to any one of claims 1 to 6, **characterized in that** the device carried by an individual is used having an additional pressure sensor, wherein
the ambient pressure, blood pressure and/or human pulse measured with said sensor are recorded in the device's memory,
said data is analysed for anomalies regarding measured ambient pressure, blood pressure and/or human pulse.

8. A method according to any one of claims 3 to 7, **characterized in that**
the device carried by an individual is used having an additional microphone and loudspeaker and a user interface, preferably in the form of a button, where the logic system of the device is capable of processing voice conversations by means of the wireless communication elements present in the device, where the user interface can be, preferable, initiated or ended by the voice call, wherein
if an anomaly is detected that could indicate a hazardous situation as a result of data analysis in any of the measurements sent to the central server by the device carried by an individual, a voice call is initiated with a person in whom the possible threat has been detected.

9. A method according to any one of claims 1 to 8, **characterized in that** the condition of people being hotel guests or employees is monitored, in particular store employees, warehouse employees, security guards, rehabilitators, elderly guardians, policemen, factory workers, especially with belt production lines.

10. A system for monitoring the condition of people for implementing a method for monitoring the condition of people according to any one of the preceding claims, **characterized in that** it comprises:
a. at least one device carried by an individual, comprising power means, logic system, memory, accelerator, preferably multi-axis, and a housing
b. a central server, preferably a computer, having means to retrieve data from a device carried by an individual,
wherein the device carried by an individual is capable of record the data by the sensors that are part of the device carried by an individual, in particular the accelerometer, and
the central server is capable of analysing data retrieved from the device carried by an individual, in particular by comparing it with reference data to detect anomalies and further disclose information about the occurrence and nature of the anomaly detected.

11. The system according to claim 10, **characterized in that** the system further comprises one or more beacons dividing the physical space into zones, wherein the device carried by an individual has means to communicate with the beacons and to record the time and identifier of said beacons in the range of which there is device carried by an individual.

12. The system according to claim 10 or 11, **characterized in that** the system further comprises means for wireless communication, preferably of WiFi, Bluetooth or BLE type, in particular routers or signal amplifiers connected to the central server, wherein the device carried by an individual has means for wireless communication with means for wireless communication, preferably of WiFi, Bluetooth or BLE type.

13. The system according to claim 10, 11 or 12, **characterized in that** the device carried by an individual additionally has one or more of the following devices: a magnetometer for detecting the setting of the device carried by an individual relative to the earth's magnetic field; a thermometer for measuring of temperature of body or human environment; pressure sensor for measuring the ambient pressure, human pulse and/or blood pressure; microphone and loudspeaker and a user interface, preferably in the form of a button, where the logic system of the device is capable of processing voice conversations using the wireless communication elements present in the device, where the user interface can preferably be initiated or ended by the voice call.

14. The system according to claim 12 or 13, **characterized in that** the logic system included in a device carried by an individual and/or a central server contain a set of predefined ranges of value of qualities measured by a device carried by an individual, in particular responding to threat situations, especially accelerations, upon detection of which the central server may generate alarm information.

15. The system according to any one of the claims 10 to 14, **characterized in that** the device carried by an individual has a substantially cuboidal housing and has seven buttons, of which:
- six buttons (5, 25) are placed in two groups of three placed in one line of the buttons (5, 25), near and along the two shorter opposite edges of the front surface of the housing (1, 21) of the device, at regular intervals, wherein the middle buttons in each group of buttons (5, 25) being at the same distance from the longer opposite edges of the front surface of the housing (1, 21) of the device
- the control button (6, 26) is placed on the front surface of the housing (1, 21) at the symmetry centre of the rectangle defined by the shorter side wall of the housing (1, 21) and the adjacent halves of the longer side walls of the housing (1, 21),
wherein the buttons (5, 25) resist pressing against an equivalent mass force of 150 g to 500 g, preferably 300 g,
the signalling diode (3, 23) is placed in the symmetry centre of the rectangle defined by the left longer side wall of the housing (1, 21) and the adjacent halves of the shorter side walls of the housing (1, 21).
